# EUROPÄISCHE PATENTSCHRIFT

(11) **EP 2 245 079 B1**
(45) Veröffentlichungstag und Bekanntmachung des Hinweises auf die Patenterteilung: **17.10.2012**
(21) Anmeldenummer: 09710541.5
(22) Anmeldetag: 06.02.2009
(51) Int. Cl.: C08G 18/73, C09J 175/12, A61L 15/58, C08G 18/50, C08G 18/32, C09J 175/04, C08G 18/10, C08G 18/12

(54) **KLEBSTOFF**
ADHESIVE
ADHÉSIF

(30) Priorität: 15.02.2008 DE 102008009408
(43) Veröffentlichungstag der Anmeldung: 03.11.2010
(73) Patentinhaber: Bayer MaterialScience AG, 51368 Leverkusen (DE)
(72) Erfinder: WINTERMANTEL, Matthias, 51465 Bergisch Gladbach (DE); KARAFILIDIS, Christos, 51375 Leverkusen (DE); LUCAS, Heinz-Werner, 51467 Bergisch Gladbach (DE); SCHMIDT, Axel, 239191 Singapore (SG)
(86) Internationale Anmeldenummer: PCT/EP2009/000817
(87) Internationale Veröffentlichungsnummer: WO 2009/100852

(56) Entgegenhaltungen:
- EP-A1- 1 167 415

## Beschreibung

Die vorliegende Erfindung betrifft die Verwendung spezieller Isocyanat-terminierter Polyurethanprepolymeren in Klebstoffforrnulierungen. Diese Klebstoffformulierungen können in Anwendungen eingesetzt werden, bei denen es im direkten oder indirekten Kontakt der Klebstoffschicht mit darauf empfindlichen Substraten auf die Vermeidung oder Minimierung von Migraten ankommt.

Empfindlichen Substrate im Sinne der vorliegenden Erfindung können beispielsweise die menschliche Haut oder auch Verbundfolien sein. Letztere werden in großem Umfang zur Herstellung von Verpackungen für Güter aller Art eingesetzt. Da durch Monofolien, Co-Extrudierte-Mehrlagenfolien oder extrusionskaschierte Folienverbünde nicht alle Anforderungen wie Transparenz/Opazität, Bedruckbarkeit, Barriereeigenschaften, Versiegelbarkeit und Mechanik abgedeckt werden können, machen Verbundfolien in denen die einzelnen Lagen mittels Klebstoff miteinander Verbunden werden den größten Anteil im Markt aus und haben damit eine immense kommerzielle Bedeutung.

Einen besonderen Stellenwert hat dabei die Herstellung von Lebensmittelverpackungen aus Verbundfolien. Da manche verwendete Lagen auf der dem Lebensmittel zugewandten Seite geringe Barriereeigenschaften gegenüber den verwendeten Klebstoffbestandteilen haben muss die etwaige Migration von Klebstoffbestandteilen in das Lebensmittel besonders beachtet werden.

Im Bereich der flexiblen Verpackungsverbundfolien werden hauptsächlich aromatische Polyurethansysteme eingesetzt. Besonders kritisch ist deshalb die Migration von aromatischen Polyisocyanaten bzw. deren Reaktionsprodukten mit Wasser in das Lebensmittel. Mit Wasser, das in fast allen Lebensmitteln enthalten ist, reagieren die Polyisocyanate unter Abspaltung von Kohlendioxid zu primären aromatischen Aminen. Da primäre aromatische Amine giftig sind, haben die Gesetzgeber Grenzwerte für die Migrate aus Lebensmittelverpackungen erlassen, die unbedingt einzuhalten sind. Aus diesem Grund müssen die zur Herstellung der Verbundfolien verwendeten Klebstoffe zum Zeitpunkt des Abpackens der Lebensmittel soweit ausreagiert sein, dass die Grenzwerte sicher unterschritten werden. Analoges gilt für die Verwendung von derartigen Systemen auf der menschlichen Haut.

Ein Teil der flexiblen Verpackungen wird nach der Befüllung mit dem Lebensmittel versiegelt und anschließend sterilisiert um alle Keime abzutöten und die Haltbarkeit des Lebensmittels zu erhöhen. Die Sterilisation wird für gewöhnlich bei Temperaturen über 100°C durchgeführt. Bei diesen Temperaturen können aromatische Isocyanate durch Rückspaltung aus den Polyurethanklebstoffen frei werden und in das Lebensmittel migrieren. Aus diesem Grund werden für die Herstellung flexibler Folienverbünde gemäß §177.1390 FDA Klebstoffformulierungen auf Basis aliphatischer Isocyanate eingesetzt. Aliphatische Polyisocyanate bilden bei der Reaktion mit Wasser naturgemäß keine primären aromatischen Amine und sind daher zur Herstellung von flexiblen Folienverbünden, die dafür vorgesehen, sind mit dem Lebensmittel eine Sterilisation zu durchlaufen sehr vorteilhaft. Wie aus der Polyurethanchemie allgemein bekannt ist, weisen aliphatische Polyisocyanate jedoch eine deutlich geringe Reaktivität gegenüber Polyolen auf als aromatische Polyisocyanate. Deshalb sind die Aushärtezeiten von aliphatischen Klebstoffformulierungen bei Raumtemperatur extrem lang, was vor der Verwendung des Folienverbundes eine lange Aushärte- und damit Lagerzeit des selbigen voraussetzt. Setzt man den Folienverbund vor der vollständigen Aushärtung dem Abpack- und Sterilisationsprozess aus, so kann dies aufgrund von noch nicht vollständig ausgebildeter Verbundhaftung zu Delamination des Folienverbundes und damit zur Zerstörung der Verpackung führen. Aus wirtschaftlichen wie auch logistischen Gründen wird versucht, die notwendige Lagerzeit bis zur vollständigen Aushärtung auf ein Minimum zu reduzieren. Dazu werden zwei verschiedene Konzepte verwendet:
1) Beschleunigung der chemischen Aushärtungsreaktion der Klebstoffformulierung durch den Zusatz von Katalysatoren.
2) Temperung der Verbundfolien unmittelbar nach der Kaschierung für 3-7 Tage bei Temperaturen von mindestens 40 °C und mehr.

So beschreibt die WO 2006/026670 beispielsweise die Verwendung eines Polurethan-Prepolymers basierend auf aliphatischen Polyisocyanaten in einer Klebstoffformulierung, die bei 60 °C in drei Tagen eine ausreichende Verbundhaftung zeigt. Dem Polyurethan-Prepolymer wird dabei zusätzlich zur erhöhten Aushärtungstemperatur ein Katalysator (Dibutylzinndilaurat, DBTL) zugesetzt. Nachteilig ist dabei zum einen die sehr hohe Aushärtetemperatur von 60°C, die aufwendige Temperkammern oder -öfen erfordert und zu Rollenteleskopierung und Faltenbildng führen kann und zum anderen der verwendete, in diesem Fall sogar schwermetallhaltige, Katalysator.

Die US-A 2006/0078741 beschreibt die Verwendung von Katalysatoren, um die Aushärtezeit von Klebstoffformulierungen für die Herstellung von Folienverbünden zu verkürzen. Die verkürzte Aushärtezeit korreliert mit der Lagerzeit des Folienverbund vor dessen Einsatz zur Verpackung von Lebensmitteln. Nachteilig bei beiden Formulierungen ist, dass der Katalysator innerhalb des Folienverbundes migrationsfähig bleibt und grundsätzlich das abgepackte Lebensmittel kontaminieren kann.

Aus der EP-A 1 167 415 sind Hydroxy-terminierte Prepolymere bekannt geworden, die mit Isocyanaten zu Beschichtungen vernetzt werden können. Die mechanischen Eigenschaften dieser Beschichtungen sind zwar im Hinblick auf die konkrete Lehre dieses Stands der Technik akzeptabel, die Vernetzungsreaktion muss jedoch katalysiert werden, und die in der Beschichtung verbleibenden toxikologisch bedenklichen Katalysatoren können in die beschichteten Substrate oder in mit diesen in Kontakt stehenden Medien migrieren, was höchst unerwünscht ist.

Aufgabe der vorliegenden Erfindung war es daher, Klebstoffformulierungen basierend auf einem aliphatischen Polyisocyanat zu entwickeln, die frei von migrationsfähigen Katalysatoren sind und dennoch bei Raumtemperatur eingesetzt werden können, so dass innerhalb von höchstens drei Tagen beispielsweise eine hinreichende Verbundhaftung in Verbundfolien erreicht wird und/oder sie bei der Herstellung von Wundverschluß- und Wundversorgungsmitteln einsetzbar sind. Eine hinreichende Verbundhaftung von Verbundfolien beträgt 3 N/15mm oder höher.

Überraschenderweise wurde nun gefunden, dass Klebstofffformulierungen basierend auf aliphatischen Polyisocyanaten innerhalb von 3 Tagen bei Raumtemperatur eine hinreichende Verbundhaftung aufbauen und dennoch keinen migrationsfähigen Katalysator enthalten, wenn man aliphatische NCO-Prepolymere einsetzt, die polymergebundene tertiäre Aminogruppen tragen.

Gegenstand der vorliegenden Erfindung sind daher zunächst Isocyanat-terminierte Prepolymere auf Basis aliphatischer Isocyanate, die an das Prepolymer gebundene tertiäre Aminogruppen enthalten.

In einer Ausführungsform der Erfindung werden diese tertiären Aminogruppen durch die Polyisocyanat-Komponente in das Prepolymer eingeführt.

In einer anderen Ausführungsform der Erfindung werden diese tertiären Aminogruppen durch die isocyanat-reaktive Komponente in das Prepolymer eingeführt.

Zur Herstellung der erfindungsgemäßen Isocyanat-terminierten Prepolymere eingesetzte aliphatische Polyisocyanate weisen bevorzugt einen NCO-Gehalt von 11-51 Gew.% und eine nominelle mittlere Funktionalität von 2 bis 3,8 auf.

Gegenstand der Erfindung sind auch Zubereitungen, die die vorstehend beschriebenen Isocyanat-terminierten Prepolymere enthalten.

Bevorzugt handelt es sich bei diesen Zubereitungen um Klebstoffe. Diese können zum allgemein zum Verkleben von Substraten eingesetzt werden, in einer bevorzugten Ausführungsform werden die Klebstoffe zum Verkleben von Verpackungsmaterialien aller Art eingesetzt, in einer besonders bevorzugten Form zur Herstellung von Folienverbünden.

Bei diesen Folienverbünden kann es sich um Klebenähte von Folien oder auch um ganzflächig verklebte Folien handeln, wie es beispielsweise bei Verbundfolien der Fall ist.

Insbesondere sind Lebensmittelverpackungen, die mit Hilfe von Klebstoffen, die auf Basis der erfindungsgemäßen Prepolymere hergestellt oder verschlossen werden ebenfalls Gegenstand der vorliegenden Erfindung. Hierbei handelt es sich bevorzugt um Verbundfolien, mit denen das Lebensmittel zum Zweck der Verpackung desselben zumindestens teilweise umhüllt wird. "Teilweise umhüllt" sind z.B. in tiefgezogenen Kunststoffverpackungsschalen eingebrachte Gegenstände, wenn diese Schalen mit einer solchen Folie verschlossen sind, ggf auch unter Verwendung erfindungsgemäßer Klebstoffe.

Die erfindungsgemäßen Prepolymere können aber auch bei der Herstellung von Klebstoff- und Pflastersystemen zur Wundschließung und -versorgung eingesetzt werden, denn auch hierbei spielt die Abwesenheit von Restmonomeren und die Anwendbarkeit bei Raumtmperatur sowie die Freiheit von migationsfähigen Bestandteilen eine bedeutende Rolle.

In einer bevorzugten Ausführungsform der vorliegenden Erfindung wird eine bei Raumtemperatur innerhalb von 3 Tagen hinreichende Verbundhaftung ausbildende Klebstoffformulierung für Verbundmaterialien bereit gestellt, enthaltend:
A) Eine Isocyanatgruppen tragenden Komponente, enthaltend mindestens ein Polyisocyanat, dadurch gekennzeichnet, dass
   a1) das Polyisocyanat
      i. eine mittlere Funktionalität im Bereich von 2 bis 3,8, bevorzugt von 2 bis 3,2, besonders bevorzugt von 2 bis 2,4 hat,
      ii. einen NCO-Gehalt im Bereich von 11 bis 51 Gew.%, bevorzugt von 21 bis 51 Gew.-%, besonders bevorzugt von 23 bis 51 Gew.% hat,
   a2) die Isocyanatgruppen tragende Komponente polymergebundene tertiäre Aminogruppen trägt;
B) Eine Polyolkomponente, mindestens enthaltend eine Polyhydroxyverbindung, dadurch gekennzeichnet, dass
   a1) die mittlere Funktionalität im Bereich von 2 bis 4, bevorzugt im Bereich von 2 bis 3,6 liegt.
   a2) die OH-Zahl im Bereich von 6 bis 720 mg KOH / g, bevorzugt im Bereich von 28 bis 480 mg KOH / g und besonders bevorzugt im Bereich von 40 bis 240 mg KOH / g liegt.
C) gegebenenfalls weitere Additive;
sowie ein Verfahren zur Herstellung von Verbundmaterialien unter Verwendung oben genannter Klebstofffortnulierung.

In einer weiteren bevorzugten Verwendung werden diese oder ähnliche erfindungsgemäße Klebstoffzubereitungen als chirurgische Klebstoffe zur Wundschließung und -versorgung bzw. bei der Herstellung von Klebstoff- und Pflastersystemen zur Wundschließung und -versorgung eingesetzt, wie sie als Pflaster beispielsweise aus der EP-A 0 897 406 bekannt sind, oder auch ohne textilen Träger direkt als Wundklebstoff bzw. Wundverschlußmittel. In diese Klebstoffzubereitungen können zusätzlich Wirkstoffe eingearbeitet werden, die das Wundverhalten positiv beeinflussen. Hierzu gehören beispielsweise antimikrobiell wirkende Agentien wie Antimycotika und antibakteriell wirkende Stoffe (Antibiotika), Corticosteroide, Chitosan, Dexpanthenol und Chlorhexidingluconat.

Daher betrifft die vorliegende Erfindung die Verwendung von aliphatischen Isocyanat-terminierten Polyurethanprepolymeren, die tertiäre Aminogruppen enthalten, in Klebstoffformulierungen für die Herstellung von Verbundfolien, die bei Raumtemperatur innerhalb von wenigen Tagen eine hinreichende Verbundhaftung aufweisen und frei von migrationsfähigen Katalysatoren sind sowie bei der Herstellung von medizinischen Wundversorgungssystemen.

Die Herstellung der in A) eingesetzten Polyisocyanat-Prepolymere ist dem Fachmann an sich bekannt und erfolgt durch Umsetzung der Polyhydroxyverbindungen mit überschüssigen Mengen von Polyisocyanaten. Als Polyisocyanat können prinzipiell alle dem Fachmann bekannten organischen aliphatischen, cycloaliphatischen, aromatischen oder heterocyclischen Polyisocyanate mit mindestens zwei Isocyanatgruppen pro Molekül sowie Gemische davon eingesetzt werden. Beispiele für geeignete aliphatische bzw. cycloaliphatische Polyisocyanate sind Di- oder Triisocyanate wie z.B. Butandiisocyanat, Pentandiisocyanat, Hexandiisocyanat (Hexamethylendiisocyanat, HDI), 4-Isocyanatomethyl-1,8-octandiisocyanat (Triisocyanatononan, TIN) oder cyclische Systeme, wie z.B. 4,4'-Methylenbis(cyclohexylisocyanat), 3,5,5-Trimethyl-1-isocyanato-3-isocyanatomethylcyclohexan (Isophorondiisocyanat, IPDI), sowie ω.ω'-Diisocyanato-1,3-dimethylcyclohexan (H₆XDI). Als aromatische Polyisocyanate können z.B. 1,5-Naphthalendiisocyanat, Diisocyanatodiphenylmethan (2,2'-, 2,4'- und 4,4'-MDI oder Mischungen daraus), Diisocyanatomethylbenzol (2,4- und 2,6-Toluylendiisocyanat, TDI), insbesondere das 2,4- und das 2,6-Isomere und technische Gemische der beiden Isomeren sowie 1,3-Bis(isocyanatomethyl)benzol (XDI) eingesetzt werden. Bevorzugt ist jedoch die Verwendung von aliphatischen Diisocyanaten, besonders bevorzugt von Hexandiisocyanat (Hexamethylendiisocyanat, HDI), 3,5,5-Trimethyl-1-isocyanato-3-isocyanatomethylcyclohexan (Isophorondiisocyanat, IPDI), sowie 1,3-Bis(isocyanatomethyl)benzol (XDI).

Überdies können aber auch die an sich bekannten Folgeprodukte der vorgenannten organischen aliphatischen, cycloaliphatischen oder heterocyclischen Polyisocyanate mit U-retdion-, Allophanat-, Biuret- und/oder Isocyanurat-Struktur eingesetzt werden.

Als Polyhydroxyverbindungen können alle dem Fachmann bekannten Verbindungen eingesetzt werden, welche eine mittlere OH-Funktionalität von mindestens 1,5 aufweisen. Dies können beispielsweise niedermolekulare Diole (z.B. 1,2-Ethandiol, 1,3- bzw. 1,2-Propandiol, 1,4-Butandiol), Triole (z.B. Glycerin, Trimethylolpropan) und Tetraole (z.B. Pentaerythrit) sein, aber auch höhermolekulare Polyhydroxyverbindungen wie Polyetherpolyole, Polyesterpolyole, Polycarbonatpolyole sowie Polythioetherpolyole. Bevorzugt weisen solche Polyetherpolyole OH-Zahlen von 5 bis 620 mg KOH/ g, bevorzugt von 14 bis 550 mg KOH / g und besonders bevorzugt von 28 bis 480 mg KOH / g auf. Solche Polyetherpolyole sind in an sich bekannter Weise durch Alkoxylierung von geeigneten Starter-Molekülen unter Basenkatalyse oder Einsatz von Doppelmetallcyanidverbindungen (DMC-Verbindungen) zugänglich. Geeignete Starter-Moleküle für die Herstellung von Polyetherpolyolen sind Moleküle mit mindestens 2 gegenüber Epoxiden reaktiven Element-Wasserstoffbindungen oder beliebige Gemische derartiger Starter-Moleküle. Bevorzugt sind Polyetherpolyolmischungen, die wenigstens ein Polyol mit wenigstens einer tertiären Aminogruppe, enthalten. Solche tertäre Aminogruppen aufweisenden Polyetherpolyole lassen sich durch Alkoxylierung von Startermolekülen oder Mischungen von Startermolekülen, wenigstens enthaltend ein Startermolekül mit mindestens 2 gegenüber Epoxiden reaktiven Element-Wasserstoffbindungen, von denen mindestens eine eine NH-Bindung ist, oder niedermolekulare Polyolverbindungen die tertiäre Aminogruppen tragen, herstellen. Beispiele für geeignete Startermoleküle sind Ammoniak, Methylamin, Ethylamin, n-Propylamin, iso-Propylamin, Ethanolamin, Diethanolamin, Triethanolamin, Ethylendiamin, Ethylentriamin, Triethanolamin, N-Methyldiethanolamin, N,N'-Dimethyl-ethylendiamin, Tetramethylendiamin, Hexamethylendiamin, 2,4-Toluylendiamin, 2,6-Toluylendiamin, Anilin, Diphenylmethan-2,2'-diamin, Diphenylmethan-2,4'-diamin, Diphenylmethan-4,4'-diamin, 1-Aminomethyl-3-amino-1,5,5-trimethylcyclohexan (Isophorondiamin), Dicyclohexylmethan-4,4'-diamin, Xylylendiamin und Polyoxyalkylenamine.

Grundsätzlich können auch Mischungen von mehreren Polyisocyanaten und/oder Polyhydroxyverbindungen eingesetzt werden, bevorzugt ist jedoch die Verwendung nur eines Polyisocyanats. Typischerweise beträgt dabei das Mol-Verhältnis der NCO-Gruppen der Polyisocyanate zu OH-Gruppen der Polyhydroxyverbindungen 25 : 1 bis 1,5 : 1, bevorzugt 20 : 1 bis 1,5 : 1 und besonders bevorzugt 15 : 1 bis 1,5 : 1. Die Umsetzung erfolgt im Allgemeinen bei Temperaturen von 20 bis 140°C, bevorzugt bei 40 bis 120°C. Grundsätzlich kann die Reaktion durch die Verwendung von aus der Polyurethanchemie an sich bekannten Katalysatoren wie beispielsweise Zinn-Seifen, z.B. Dibutylzinndilaurat oder tertiären Aminen, z.B. Triethylamin oder Diazabicyclooctan (DABCO) beschleunigt werden, diese Verfahrensweise ist aber nicht bevorzugt. Die Zugabe der Komponenten und gegebenenfalls eines Katalysators der vorgenannten Art kann grundsätzlich in beliebiger Reihenfolge erfolgen. Wird das Polyisocyanat im Überschuss eingesetzt, so ist bevorzugt, dieses nach der Umsetzung durch Extraktion oder Destillation, vorzugsweise mittels Dünnschichtdestillation, abzutrennen. Die Abtrennung des überschüssigen Polyisocyanats wird dabei soweit geführt, das im resultierenden Polyisocyanat-Prepolymer weniger als 1 Gew.%, bevorzugt weniger als 0,5 Gew.% und besonders bevorzugt weniger als 0,2 Gew.% des Polyisocyanats verbleibt.

Als Polyhydroxyverbindungen in B) können alle dem Fachmann bekannten Verbindungen eingesetzt werden, welche eine mittlere OH-Funktionalität von mindestens 1,5 aufweisen. Dies können beispielsweise niedermolekulare Diole (z.B. 1,2-Ethandiol, 1,3- bzw. 1,2 Propandiol, 1,4-Butandiol), Triole (z.B. Glycerin, Trimethylolpropan) und Tetraole (z.B. Pentaerythrit) sein, aber auch höhermolekulare Polyhydroxyverbindungen wie Polyetherpolyole, Polyesterpolyole, Polycarbonatpolyole sowie Polythioetherpolyole. Bevorzugt sind jedoch solche Polyesterpolyole, die eine Hydroxylzahl von 6 bis 720 mg KOH/g, bevorzugt von 28 bis 480 mg KOH/g und besonders bevorzugt von 40 bis 240 mg KOH/g aufweisen und eine mittlere Funktionalität von 2 bis 4, bevorzugt 2 bis 3,7 und besonders bevorzugt von 2 bis 3,6 besitzen. Solche Polyesterpolyole können in bekannter Weise durch Polykondensation von niedermolekularer Polycarbonsäurederivaten, wie beispielsweise Bernsteinsäure, Adipinsäure, Korksäure, Azelainsäure, Sebacinsäure, Dodecandisäure, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Dimerfettsäure, Trimerfettsäure, Phthalsäure, Phthalsäureanhydrid, Isophthalsäure, Terephthalsäure, Zitronensäure oder Trimellithsäure, mit niedermolekularen Polyolen, wie beispielsweise Ethylenglykol, Diethylenglykol, Neopentylglykol, Hexandiol, Butandiol, Propylenglykol, Glycerin, Trimethylolpropan 1,4-Hydroxymethylcyclohexan, 2-Methyl-1,3-propandiol, Butantriol-1,2,4, Triethylenglykol, Tetraethylenglykol, Polyethylenglykol, Dipropylenglykol, Polypropylenglykol, Dibutylenglykol und Polybutylenglykol, oder durch Ring öffnende Polymerisation cyclischer Carbonsäureester, wie ε-Caprolacton, herstellen. Darüberhinaus lassen sich auch Hydroxycarbonsäurederivate, wie beispielsweise Milchsäure, Zimtsäure oder ω-Hydroxycapronsäure zu Polyesterpolyolen polykondensieren. Es können aber auch Polyesterpolyole oleochemischer Herkunft verwendet werden. Derartige Polyesterpolyole können beispielsweise durch vollständige Ringöffnung von epoxidierten Triglyceriden eines wenigstens teilweise olefinisch ungesättigte Fettsäure-enthaltenden Fettgemisches mit einem oder mehreren Alkoholen mit 1 bis 12 C-Atomen und anschließender partieller Umesterung der Triglycerid-Derivate zu Alkylesterpolyolen mit 1 bis 12 C-Atomen im Alkylrest hergestellt werden.

Die Komponenten aus A) und B) werden in einem molaren Verhältnis von IsocyanatGruppen zu Hydroxyl-Gruppen von 1:1 bis zu 1,8:1, bevorzugt in einem molaren Verhältnis von Isocyanat-Gruppen:Hydroxyl-Gruppen von 1:1 bis zu 1,6:1 und besonders bevorzugt in einem molaren Verhältnis von Isocyanat-Gruppen:Hydroxyl-Gruppen von 1,05:1 bis zu 1,5:1 miteinander gemischt.

Als Additive C) kann die Klebstoffformulierung neben den vorstehend genannten Komponenten zusätzlich noch aus der Klebstofftechnologie bekannte Additive als Formulierhilfsmittel enthalten. Solche Additive sind beispielsweise die üblichen Weichmacher, Füllstoffe, Pigmente, Trockenmittel, Lichtschutzmittel, Antioxidantien, Thixotropiermittel, Haftvermittler und gegebenenfalls weitere Hilfs- und Zusatzstoffe.

Als geeignete Füllstoffe seien beispielhaft Ruß, Fällungskieselsäuren, pyrogene Kieselsäuren, mineralische Kreiden und Fällungskreiden genannt.

Geeignete Weichmacher sind beispielsweise Phthalsäureester, Adipinsäureester, Alkylsulfonsäureester des Phenols oder Phosphorsäureester.

Als Thixotropiermittel seien beispielhaft pyrogene Kieselsäuren, Polyamide, hydrierte Rizinusöl-Folgeprodukte oder auch Polyvinylchlorid genannt.

Geeignete Trockenmittel sind insbesondere Alkoxysilylverbindungen, wie z.B. Vinyltrimethoxysilan, Methyltrimethoxysilan, Methyltriethoxysilan, i-Butyltrimethoxysilan, i-Butyltriethoxysilan, Octyltriethoxysilan, Octyltrimethoxysilan, Propyltriethoxysilan, Propyltrimethoxysilan, Hexadecyltrimethoxysilan, sowie anorganische Stoffe wie z.B. Calciumoxid (CaO) und Isocyanatgruppen tragende Verbindungen wie z.B. Tosylisocyanat.

Als Haftvermittler werden die bekannten funktionellen Silane eingesetzt, wie beispielsweise Aminosilane der vorstehend genannten Art, aber auch N-Aminoethyl-3-aminopropyl-trimethoxysilan, N-Aminoethyl-3-aminopropyl-methyl-dimethoxysilan, N-Aminoethyl-3-aminopropyl-trimethoxysilan, 3-Glyxidoxypropyltrimethoxysilan, 3-Glyxidoxypropyltriethoxysilan, Mercaptosilane, Bis(3-triethoxysilylpropyl)amin, Bis(3-trimethoxy-silylpropyl)amin, Oligoaminosilane, 3-Aminopropylmethyldiethoxysilan, 3-Aminopropyltriethoxysilan, triaminofunktionelles Propyltrimethoxysilan, N-(n-Butyl)-3-aminopropyltrimethoxysilan, Phenyltriethoxysilan, Phenyltrimethoxysilan, polyetherfunktionelle Trimethoxysilane und 3-Methacryloxypropyltrimethoxysilan.

Die Herstellung der Klebstoffformulierung aus der Isocyanatgruppen tragenden Komponente A) und dem Polyol bzw. der Polyolmischung B) zur Herstellung eines Folienverbundes ist dem Fachmann aus der Polyurethanchemie an sich bekannt. Die Additive C) können dem Polyol bzw. der Polyolformulierung B) oder aber der Isocyanatgruppen tragenden Komponente A) oder beiden zugesetzt werden. Bevorzugt werden die Additive C) dem Polyol bzw. der Polyolformulierung B) zugesetzt.

In einer bevorzugten Ausfiihrungsform der Erfindung werden die beiden Komponenten A) und B) der Klebstoffformulierung unmittelbar vor der Herstellung des Folienverbundes miteinander vermischt und in die Kaschiermaschine bzw. das Auftragswerk eingeführt. In einer weiteren bevorzugten Ausführungsform kann die Vermischung der Komponenten A) und B) in der Kaschiermaschine selbst unmittelbar vor dem oder im Auftragswerk geschehen. Die Klebstoffformulierung kann dabei Lösemittelfrei oder aber in einem geeigneten Lösemittel oder Lösemittelgemisch eingesetzt werden. Geeignete Lösemittel sind solche, die eine hinreichende Löslichkeit der Polyhydroxykomponente und der Polyisocyanatkomponente aufweisen. Beispiele für solche Lösemittel sind Benzol, Toluol, Ethylacetat, Butylacetate, Propylacetate, Methylethylketon, Methylisobutylketon, 2-Methoxypropylacetat. Besonders bevorzugt sind Ethylacetat und Methylethylketon. Im Auftragswerk wird die so genannte Trägerfolie mit der Klebstoffformulierung mit einem mittleren Trockenauftragsgewicht von 1 bis 9 g/m² beschichtet und durch in Kontakt bringen mit einer zweiten Folie zu dem resultierenden Folienverbund kaschiert. Gegenbenenfalls eingesetzte Lösemittel oder Lösemittelgemische werden vor dem in Kontakt bringen der Trägerfolie mit der zweiten Folien vollständig in einem Trockenkanal oder in einer anderen geeigneten Vorrichtung entfernt.

Die Klebstoffformulierung wird bevorzugt zum Verkleben von Kunststofffolien, Aluminiumfolien, anderen Metallfolien, Metall-bedampften Kunststofffolien und Metalloxidbedampften Kunststofffolien verwendet.

Die Erfindung wird durch die nachfolgenden nicht limitierenden Beispiele erläutert.

### Beispiele:

In den nachfolgenden Beispielen beziehen sich die Prozentangaben auf das Gewicht. Die Viskositäten wurden falls nicht anders angegeben bei einer Messtemperatur von 25°C mit Hilfe des Rotationsviskosimeters Viskotester VT 550 der Fa. Thermo Haake, Karlsruhe, DE mit dem Messbecher SV und der Messeinrichtung SV DIN bestimmt. Die Bestimmung des NCO-Gehalts der Prepolymere bzw. Reaktionsmischungen erfolgte nach DIN EN 1242.

### Folgende Abkürzungen wurden verwendet:

- OHZ:: Hydroxylzahl [mg KOH/g]
- SZ:: Säurezahl [mg KOH/g]
- %NCO:: NCO-Gehalt in Gew.-% NCO-Gruppen
- VH:: Verbundhaftung [N/15mm] zwischen der Aluminium und der Polyethylen Lage im folgenden Verbund 12 µm Polyethylenterephthalat / 9 µm Aluminiumfolie / 60 µm Polyethylenfolie

### Abkürzungen der verwendeten Einsatzstoffe:

### Polyole:

- P1:: Polypropylenethertetraol gestartet mit Ethylendiamin, hergestellt mittels KOH-Katalyse, OHZ 470.
- P2:: Polypropylenetherdiol gestartet mit 1,2-Propylenglykol, hergestellt mittels KOH-Katalyse, OHZ 262.
- P3:: Polypropylenetherdiol gestartet mit 1,2-Propylenglykol, hergestellt mittels KOH-Katalyse, OHZ 112.
- P4:: Polyesterpolyol als Reaktionsprodukt aus 14 Gew.-Teilen Adipinsäure, 39 Gew.-Teilen Isophthalsäure, 7 Gew.-Teilen Phthalsäureanhydrid, 12 Gew.-Teilen Trimethylolpropan und 42 Gew.-Teilen 1,6-Hexandiol, OHZ 141, SZ ≤ 3.
- P5:: Polyesterpolyol als Reaktionsprodukt aus 11,5 Gew.-Teilen Adipinsäure, 32,9 Gew.-Teilen Isophthalsäure, 5,9 Gew.-Teilen Phthalsäureanhydrid, 13,4 Gew.-Teilen Trimethylolpropan und 47,0 Gew.-Teilen 1,6-Hexandiol, OHZ 242, SZ ≤ 3.

### Polyisocyanate:

### NCO1 :

Hexamethylen-1,6-diisocyanat (HDI) mit einem Gehalt ≥ 99,5 Gew.-% und ≥ 49,7 %NCO.

### Erfindungsgemäßes tert. Aminogruppen aufweisendes aliphatisches Prepolymer:

Zu 8757g NCO1 tropft man bei 100°C und unter. ständigem Rühren innerhalb von 2 Stunden 1242g P1. Nach vollständigem Umsatz wird der Überschuss HDI bei 130°C und <1mbar destillativ abgetrennt. Man erhält ein Prepolymer mit den folgenden Kenndaten: Viskosität (23°C, 40s⁻¹) 139Pas; 15,0 %NCO, 0,18 Gew.% freies HDI.

### Nicht erfindungsgemäßes tert. Aminogruppen-freies aliphatisches Prepolymer A:

Zu 2137 g NCO1 tropft man bei 100°C und unter ständigem Rühren innerhalb von 2 Stunden 363g P2. Nach vollständigem Umsatz wird der Überschuss HDI bei 130°C und <1mbar destillativ abgetrennt. Man erhält ein Prepolymer mit den folgenden Kenndaten: Viskosität (23°C, 40s⁻¹) 1054mPas; 10,91%NCO, 0,04 gew% freies HDI.

### Nicht erfindungsgemäßes tert. Aminogruppen-freies aliphatisches Prepolymer B:

Zu 3936 g NCO1 tropft man bei 100°C und unter ständigem Rühren innerhalb von 2 Stunden 2563g P3. Nach vollständigem Umsatz wird der Überschuss HDI bei 130°C und <1mbar destillativ abgetrennt. Man erhält ein Prepolymer mit den folgenden Kenndaten: Viskosität (23°C, 40s⁻¹) 1262mPas; 6,49 %NCO, 0,03gew% freies HDI.

### Ansetzen der Klebstoffformulierung:

Da die Mischung der Polyolkomponente und der Polyisocyanatkomponente naturgemäß nicht lagerfähig ist wird diese unmittelbar vor der Herstellung des Folienverbundes hergestellt.

Die Klebstoffformulierung wird hergestellt, durch inniges Vermischen der Polyolkomponente und der Polyisocyanatkomponente. Die Mischung wird mit einem 1,4-fachen molaren Überschuss an Isocyanatgruppen hergestellt und sofort verarbeitet.

### Herstellung der Folienverbunde mittels der in Tabelle 1 beschriebenen Klebstoff formulierungen:

Die Herstellung der Folienverbunde erfolgt durch eine "Polytest 440" Lösemittelfreie-Kaschieranlage der Fa. Polytype in Freiburg, Schweiz.

Die Folienverbunde werden aus einem Polyethylenterephthalat/Aluminium Vorverbund und einer Polyethylenfolie hergestellt. Dabei wird die Aluminium-Seite des Vorverbundes mit der Klebstoffformulierung bestrichen, mit der Polyethylenfolie verklebt und anschließend auf einen Rollenkern aufgewickelt. Die Länge des mit der Klebstoff formulierung hergestellten Folienverbundes beträgt mindestens 20 m. Die trockene Auftragsmenge der Klebstoffformulierung liegt zwischen 1,9 g und 2,8 g und die Walzentemperatur des Auftragswerkes bei 30-40 °C.

**Tabelle 1: Ansatzmengen und Ausprüfung der Klebstoffformulierungen:**

| Einsatzstoffe in Gew.-% | Klebstoffformulierung erfindungsgemäß | Klebstoffformulierung nicht erfindungsgemäß | | |
|---|---|---|---|---|
| | 1 | a | b | c |
| erfindungsgemäßes tertiäre Aminogruppen aufweisendes Prepolymer | 49,6 | | | |
| nicht erfindungsgemäßes tertiäre aminogruppen-freies Prepolymer A | | 69,9 | | |
| nicht erfindungsgemäßes tertiäre aminogruppen-freies Prepolymer B | | | 69,9 | 69,9 |
| P4 | 50,4 | | 30,1 | 30,1 |
| P5 | | 30,1 | | |
| VH nach x d bei 23 °C | | | | |
| 1 | 2,3 | 0,0 | 0,0 | 1,3 |
| 2 | 4,3 | 0,2 | 0,0 | 1,4 |
| 3 | 4,7 | 2,6 | 0,7 | 1,2 |
| 7 | 4,3 | 2,0 | 1,2 | 1,5 |
| 14 | 3,6 | 1,7 | 1,2 | 1,2 |
| 28 | 3,6 | 1,6 | 1,5 | 1,5 |

## Patentansprüche

1. Isocyanat-terminierte Prepolymere auf Basis aliphatischer Isocyanate, die an das Prepolymer gebundene tertiäre Aminogruppen enthalten.

2. Isocyanat-terminierte Prepolymere gemäß Anspruch 1, **dadurch gekennzeichnet, dass** die tertiären Aminogruppen durch die Polyisocyanat-Komponente in das Präpolymer eingeführt werden.

3. Isocyanat-terminierte Prepolymere gemäß Anspruch 1, **dadurch gekennzeichnet, dass** die tertiären Aminogruppen durch die isocyanat-reaktive Komponente in das Präpolymer eingeführt werden.

4. Isocyanat-terminierte Prepolymere gemäß Anspruch 1, **dadurch gekennzeichnet, dass** das zu ihrer Herstellung eingesetzte aliphatische Polyisocyanat einen NCO-Gehalt von 11-51 Gew.% und eine nominelle mittlere Funktionalität von 2 bis 3,8 aufweist.

5. Isocyanat-terminierte Prepolymere gemäß Ansprüchen 1 bis 4 enthaltende Zubereitungen.

6. Zubereitungen gemäß Anspruch 5, **dadurch gekennzeichnet, dass** es sich um Klebstoffe handelt.

7. Verwendung von Zubereitungen gemäß Ansprüchen 5 und 6 bei der Herstellung von Klebstoff- und Pflastersystemen zur Wundschließung und -versorgung.

8. Mit Zubereitungen gemäß Ansprüchen 4 und 6 beschichtete Substrate.

9. Substrate gemäß Anspruch 8, **dadurch gekennzeichnet, dass** es sich um Verpackungsmaterialien handelt.

10. Verwendung von Klebstoffen gemäß Anspruch 6 zur Herstellung von Folienverbünden.

11. Verwendung gemäß Anspruch 10, **dadurch gekennzeichnet, dass** es sich um Verbundfolien handelt.

12. Verwendung gemäß Ansprüchen 10 und 11, **dadurch gekennzeichnet, dass** es sich um Folien zur Lebensmittelverpackung handelt.

13. Mit Substraten gemäß Anspruch 8 zumindest teilweise verpackte Lebensmittel.

## Claims

1. Isocyanate-terminated prepolymers based on aliphatic isocyanates, comprising tertiary amino groups bonded to the prepolymer.

2. Isocyanate-terminated prepolymers according to Claim 1, **characterized in that** the tertiary amino groups are introduced into the prepolymer by the polyisocyanate component.

3. Isocyanate-terminated prepolymers according to Claim 1, **characterized in that** the tertiary amino groups are introduced into the prepolymer by the isocyanate-reactive component.

4. Isocyanate-terminated prepolymers according to Claim 1, **characterized in that** the aliphatic polyisocyanate used for their preparation has an NCO content of 11-51% by weight and a nominal average functionality of 2 to 3.8.

5. Preparations comprising isocyanate-terminated prepolymers according to Claims 1 to 4.

6. Preparations according to Claim 5, **characterized in that** they are adhesives.

7. Use of preparations according to Claims 5 and 6 in the production of adhesive systems and plaster systems for wound closure and wound management.

8. Substrates coated with preparations according to Claims 5 and 6.

9. Substrates according to Claim 8, **characterized in that** they are packaging materials.

10. Use of adhesives according to Claim 6 for producing film composites.

11. Use according to Claim 10, **characterized in that** the composites are composite films.

12. Use according to Claims 10 and 11, **characterized in that** the films are films for food packaging.

13. Foods packaged partly at least with substrates according to Claim 8.

## Revendications

1. Prépolymères à terminaison isocyanate, à base d'isocyanates aliphatiques, qui contiennent des groupes amino tertiaires liés au prépolymère.

2. Prépolymères à terminaison isocyanate selon la revendication 1, **caractérisés en ce que** les groupes amino tertiaires sont introduits dans le prépolymère par le composant polyisocyanate.

3. Prépolymères à terminaison isocyanate selon la revendication 1, **caractérisés en ce que** les groupes amino tertiaires sont introduits dans le prépolymère par le composant réactif avec un isocyanate.

4. Prépolymères à terminaison isocyanate selon la revendication 1, **caractérisés en ce que** le polyisocyanate aliphatique utilisé pour leur préparation présente une teneur en NCO de 11-51 % en poids et une fonctionnalité moyenne nominale de 2 à 3,8.

5. Préparations contenant des prépolymères à terminaison isocyanate selon les revendications 1 à 4.

6. Préparations selon la revendication 5, **caractérisée en ce qu'**il s'agit d'adhésifs.

7. Utilisation de préparations selon les revendications 5 et 6, dans la fabrication de systèmes d'adhésif et pansement pour la fermeture et le soin des plaies.

8. Supports revêtus avec des préparations selon les revendications 5 et 6.

9. Supports selon la revendication 8, **caractérisés en ce qu'**il s'agit de matériaux d'emballage.

10. Utilisation d'adhésifs selon la revendication 6, pour la production de composites de films.

11. Utilisation selon la revendication 10, pour la production de films composites.

12. Utilisation selon les revendications 10 et 11, **caractérisée en ce qu'**il s'agit de films pour l'emballage de produits alimentaires.

13. Produits alimentaires au moins partiellement emballés avec des supports selon la revendication 8.
